(19) Europäisches Patentamt
European Patent Office
Office européen des brevets

(11) **EP 4 306 100 A1**

(12) **EUROPEAN PATENT APPLICATION**
published in accordance with Art. 153(4) EPC

(43) Date of publication:
**17.01.2024 Bulletin 2024/03**

(21) Application number: **22767498.3**

(22) Date of filing: **08.03.2022**

(51) International Patent Classification (IPC):
*A61K 8/368* (2006.01)     *A61K 8/44* (2006.01)
*A61Q 19/00* (2006.01)     *A61Q 17/00* (2006.01)

(52) Cooperative Patent Classification (CPC):
**A61K 8/368; A61K 8/44; A61Q 17/00; A61Q 19/00**

(86) International application number:
**PCT/KR2022/003302**

(87) International publication number:
**WO 2022/191602 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2021  KR 20210030240
02.12.2021  KR 20210170889**

(71) Applicant: **LG Household & Health Care Ltd.
Seoul 03184 (KR)**

(72) Inventors:
• **KWON, Koo Chul
Seoul 07795 (KR)**
• **WON, Jong Gu
Seoul 07795 (KR)**

(74) Representative: **Goddar, Heinz J.
Boehmert & Boehmert
Anwaltspartnerschaft mbB
Pettenkoferstrasse 22
80336 München (DE)**

(54) **COSMETIC COMPOSITION COMPRISING CARNITINE-SALICYLATE AS ACTIVE INGREDIENT**

(57)     The present invention relates to a cosmetic composition comprising carnitine-salicylate (CA-SA) as an active ingredient. In addition, the composition of the present invention, as a result of having CA-SA as the active ingredient, has been confirmed to alleviate acne and exfoliate dead skin cells as a skin improving substance while simultaneously alleviating inflammation and erythema as a skin soothing substance, due to ion pairing of carnitine and salicylate in mildly acidic conditions.

【Figure 1a】

EP 4 306 100 A1

**Description**

[Technical field]

The present invention relates to a cosmetic composition including carnitine-salicylate (CA-SA) as an active ingredient.

[Background Art]

Skin aging is a process in which gradual and continuous physiological changes occur due to external environmental exposure (extrinsic aging) and genetic causes (intrinsic aging). In particular, since skin tissue among various tissues of the human body is always exposed to sunlight, the death of skin epidermal stem cells is induced or pigmentation is induced by aging of melanocytes, and in the dermal layer, degradation of dermal constituents associated with skin wrinkles, such as collagen and elastin, is induced. In addition, external stimuli such as sunlight induce inflammation through the promotion of cytokine secretion, causing discomfort such as erythema, stinging, itching, and the like, as well as accelerating skin aging. Various materials for alleviating skin diseases and aging caused by such external stimuli have been used in cosmetics. For example, materials such as retinoid, hydroquinone, L-ascorbic acid, and α-hydroxy acid (AHA) are known to have an anti-aging effect, and materials such as D-panthenol and glycyrrhizic acid are known to have an anti-inflammation effect. In particular, salicylate, known as β-hydroxy acid (BHA), is known to have been used since 1550 B.C. in Egypt as a material having both anti-aging and anti-inflammation effects. In particular, it has been in the spotlight as a cosmetic ingredient because of its bactericidal effect against *Propionibacterium acnes.* However, salicylate is acidic with a pH of 3.0 or less in aqueous solutions, and may cause irritation when repeatedly applied to the skin. Accordingly, the Cosmetic Ingredient Review Expert Panel recommends using salicylate in the form of salicylate at pH 3.5 or more, and recommends applying salicylate at 3.0% or less in cosmetics. However, when salicylate is applied in an ionized form by a weak acid system, it is known to have a limitation in that skin permeability and efficacy are lowered, so that it is difficult to feel high efficacy without skin irritation. In addition, since salicylate has low solubility in weakly acidic water, in the related art, the following methods were devised: i) changing it into a salt form, ii) derivatizing it, or iii) dissolving it in strongly acidic water and then neutralizing it into a weak acid, but when the above three methods are applied, there is a problem that the original bactericidal effect of salicylate cannot be fully exerted.

An external skin preparation including carnitine and showing an acne improvement effect is disclosed (Korean Patent Publication No. 10-2010-0095447), a method of incorporating salicylic acid into a hydroxyalkylated cyclodextrin to improve its solubility in water and blending it into a cosmetic composition at a high content is known (Korean Registered Patent No. 10-2307186), and it is known that a cosmetic composition containing a salicylic acid derivative inhibits tyrosinase activity to exhibit a skin whitening effect (Korean Patent Publication No. 10-2017-0076091).

However, the related art has a problem in that the process is complicated and it is difficult to maintain the unique efficacy of each component in terms of purpose and effect.

[Related Art Documents]

1. Korean Patent Publication No. 10-2010-0095447
2. Korean Registered Patent No. 10-2307186
3. Korean Patent Publication No. 10-2017-0076091.

[Disclosure]

[Technical Problem]

Under the above background, the present inventors confirmed that a composition of the present invention, as a result of having CA-SA as an active ingredient, alleviates acne and exfoliates dead skin cells as a skin improving material while simultaneously alleviating inflammation and erythema as a skin soothing material, due to ion pairing of carnitine and salicylate in mildly acidic conditions, and completed the present invention.

Accordingly, the present invention is directed to providing a cosmetic composition including carnitine-salicylate (CA-SA) as an active ingredient.

[Technical Solution]

As a means for solving the above problems, the present invention provides a cosmetic composition comprising carnitine-salicylate (CA-SA) as an active ingredient.

In order to achieve the above object, the present invention provides a method for alleviating inflammation and erythema for skin soothing while exhibiting acne relief and exfoliation effects for skin improvement, including applying a cosmetic composition comprising carnitine-salicylate (CA-SA) as an active ingredient to the skin.

In order to achieve the above object, the present invention provides a method for alleviating inflammation and erythema for skin soothing while exhibiting acne relief and exfoliation effects for skin improvement, including administering a cosmetic composition comprising carnitine-salicylate (CA-SA) as an active ingredient to a subject.

In order to achieve the above object, the present invention provides a use of a composition comprising carnitine-salicylate (CA-SA) as an active ingredient for the preparation of cosmetics for alleviating inflammation and erythema for skin soothing while exhibiting acne relief and exfoliation effects for skin improvement

Hereinafter, the configuration of the present invention will be described in detail.

The present invention relates to a cosmetic composition comprising carnitine-salicylate (CA-SA) as an active ingredient.

In the present invention, the carnitine is 4-trimethylamino-3-hydroxybutyric acid, and may include L-carnitine, DL-carnitine, hydrochlorides thereof, and derivatives thereof. Preferably L-carnitine is used.

Carnitine has been reported as a hypoallergenic amino acid exfoliating agent that exhibits higher efficacy than $\alpha$-hydroxy acid (AHA) under weakly acidic and neutral conditions. In addition, since it has an ammonium group in its structure, it is advantageous for skin penetration and facilitates penetration into the stratum corneum.

An external skin preparation including carnitine and exhibiting an acne improvement effect is disclosed (Korean Patent Publication No. 10-2010-0095447). However, the related art only uses carnitine as an additional additive as a ceramide synthesis promotor, and the mixing ratio thereof is different, and it is not included as a major component for the major effect. In addition, though the acne bacillus test and sensory evaluation test results are described for the acne improvement effect, objective results based on standard test methods for cytotoxicity, exfoliation, inflammation relief and erythema relief efficacy of external skin preparations are not presented.

In one embodiment of the present invention, as a result of preparing carnitine-salicylate (CA-SA) and conducting *in vitro* and *in vivo* efficacy tests, the material showed little cytotoxicity, so it may exhibit acne relief and exfoliation efficacy as a skin improvement material without irritating the skin, and at the same time the inflammation and erythema relief effect as a skin soothing material may be maximized.

Salicylic acid, which is commonly used, has low solubility in water, so it is difficult to apply it in high concentrations to cosmetics, and application formulations are limited and may cause skin irritation. Therefore, it is recommended to use it in the form of salicylate, but this also has limitations in that skin permeability and efficacy are lowered when applied in weak acid systems.

A method of incorporating salicylic acid into a hydroxyalkylated cyclodextrin to improve its solubility in water and including it into a cosmetic composition at a high content is known (Korean Registered Patent No. 10-2307186), and it is also known that a cosmetic composition containing a certain salicylic acid derivative may inhibit tyrosinase activity to exhibit a skin whitening effect (Korean Patent Publication No. 10-2017-0076091). However, the related art suggests a cosmetic composition including salicylic acid as a derivative. In addition, only tyrosinase inhibition results and sensory evaluation test results are described for skin improvement effects, and objective results based on standard test methods regarding the cytotoxicity, acne relief, exfoliation, inflammation relief and erythema relief efficacy of external preparations have not been presented.

In one embodiment of the present invention, as a result of preparing carnitine-salicylate (CA-SA) and conducting *in vitro* and *in vivo* efficacy tests, the material showed little cytotoxicity, so it may exhibit acne relief and exfoliation efficacy as a skin improvement material without irritating the skin, and at the same time the inflammation and erythema relief effect as a skin soothing material may be maximized.

In the present invention, a eutectic mixture may be prepared and used in the form of carnitine-salicylate (CA-SA), in which salicylic acid is ion-paired with carnitine, instead of using salicylic acid in the form of a salt. By including carnitine-salicylate (CA-SA), the cosmetic composition according to the present invention may be prepared under weakly acidic conditions, and may exhibit more improved effects than the original efficacy of each component. Specifically, the skin improvement effect can be improved by 120% or more, or 140% or more, or 160% or more compared to a simple mixture. In addition, the cosmetic composition according to the present invention may improve the stability of the composition by including carnitine-salicylate (CA-SA). In addition, since the material has little cytotoxicity, it shows acne relief and exfoliation efficacy as a skin improvement material without irritating the skin, and at the same time, maximizes an inflammation and erythema relief effect as a skin soothing material.

The eutectic mixture means a mixture of two or more solid or liquid substances, and means that two components with high melting points are mixed and form a complex by dipole-dipole attraction between polar molecules, dipole-induced dipole attraction, hydrogen bonding between molecules, or van der Waals interactions. In other words, the bonding of the eutectic mixture refers to the bonding between molecules (formation of eutectic mixture) in a state in which the unique molecular structure of each individual is maintained based on hydrogen bonding and bonding between dipoles by the partial charge of molecules.

In addition, the eutectic mixture may be formed by combining polar compounds. The polar compound may be a polar molecule or a charged molecule. In addition, there needs to be a difference in polarity between the polar compounds, and the same compounds cannot form a eutectic mixture.

In the eutectic mixture, the ratio between molecules required for the eutectic mixture may be determined according to the charge of the molecules of the components constituting the eutectic mixture, the type and number of functional groups of the molecules, and the polarity of the molecules or functional groups. In addition, a ratio between molecules required for the eutectic mixture may be determined according to the size or similarity of molecules.

In the present invention, for carnitine-salicylate (CA-SA), the ratio between molecules required for the eutectic mixture was determined according to the charge and functional group of carnitine and salicylate molecules, and the polarity of the molecule or functional group. The carnitine-salicylate (CA-SA) may be mixed in a carnitine : salicylate molar ratio of 1 to 6:2, for example, 6:2, 5:2, 4:2, 3:2, 2:2, 1:2, 5.5:2, 4.5:2, 3.5:2, 2.5:2, or 1.5:2. It was confirmed, in Experimental Example 1-1, that carnitine-salicylate (CA-SA) was stable within the above molar ratio range.

Carnitine and salicylate can form a eutectic mixture due to bonding between dipoles by the partial charge of a specific functional group, and may form a eutectic mixture at a molar ratio within the above range through their chemical structures.

When the molar ratio is out of the above range, carnitine has degraded low-temperature stability, and salicylate may be precipitated at a low temperature due to remaining salicylate molecules that cannot ion-pair with carnitine, thatis, as their water solubility is exceeded.

In the present invention, the carnitine-salicylate (CA-SA) may preferably be in a transparent phase without precipitate when left at a low temperature. More specifically, no precipitate may be formed even when left at 0 °C for 3 weeks, 4 weeks, 5 weeks, or 6 weeks or more.

The cosmetic composition of the present invention may be in the form of a general emulsified formulation and a solubilized formulation. For example, the cosmetic composition may have formulations of a toner such as a skin softening toner or a nutritional skin toner, a lotion such as a facial lotion or a body lotion, a cream such as a nourishing cream, a moisturizing cream, or an eye cream, an essence, a cosmetic ointment, a balm, a spray, a gel, a pack, a sunscreen, a makeup base, a liquid-, solid-, or spray-type foundation, a powder, a makeup remover such as a cleansing cream, a cleansing lotion, or a cleansing oil, a cleanser such as cleansing foam, soap, body wash, and the like.

In addition, the cosmetic may contain, in addition to the composition of the present invention, adjuvants commonly used in cosmetology, such as fatty materials, organic solvents, solubilizing agents, thickening agents, gelling agents, softening agents, antioxidants, suspending agents, stabilizers, foaming agents, fragrances, surfactants, water, ionic or nonionic emulsifiers, fillers, sequestering and chelating agents, preservatives, vitamins, blocking agents, wetting agents, essential oils, dyes, pigments, hydrophilic or lipophilic active agents, lipid vesicles, or any other ingredient commonly used in cosmetics.

The cosmetic formulation may include a relatively high concentration of the composition in a wash-off type cosmetic such as a make-up remover, cleanser, and the like in which active ingredients remain on the skin for a short period of time. On the other hand, in leave-on type cosmetics such as toners, lotions, creams, essence, and the like in which active ingredients remain on the skin for a long time, the cosmetics may also include a low concentration of the composition compared to wash-off type cosmetics. Although not limited thereto, in one embodiment of the present invention, the composition of the present invention may include 0.01 to 20 parts by weight, for example, 0.01 to 20 parts by weight, 0.01 to 1.2 parts by weight, 0.01 to 1.8 parts by weight, 0.01 to 2.5 parts by weight, 0.01 to 4.7 parts by weight, 0.1 to 7.3 parts by weight, 0.01 to 10 parts by weight, 1.2 to 20 parts by weight, or 1.8 to 10 parts by weight of carnitine-salicylate (CA-SA), based on the total weight of the composition.

When the carnitine salicylate (CA-SA) according to the present invention is included in an amount less than 0.01 part by weight based on the total weight of the composition, sufficient acne relief, exfoliation promotion, inflammation relief and erythema relief effects cannot be expected, and when it is included in an amount of more than 20 parts by weight, there may be unwanted reactions such as allergies or problems with skin safety.

Each of the above components included in the cosmetic composition according to the present invention may be included in the cosmetic composition of the present invention within a range that does not exceed the maximum amount prescribed in each country's cosmetic safety standards.

In addition, the present invention provides a method of preparing a cosmetic composition comprising carnitine-salicylate (CA-SA).

Hereinafter, the configuration of the preparation method of the present invention will be described in detail.

The preparation method of the present invention may comprise preparing a mixture of carnitine and salicylic acid; stirring the mixture of the carnitine and salicylic acid at 50 °C to 70 °C to prepare carnitine-salicylate (CA-SA); and

adding the carnitine-salicylate (CA-SA) to prepare a cosmetic composition.

The preparing of the mixture of carnitine and salicylic acid may include adding purified water.

In the preparing of the carnitine-salicylate (CA-SA), when the temperature is less than 50 °C, eutectic bonding may not be sufficiently performed, and when the temperature exceeds 70 °C, the eutectic bonding may be broken and the effect of the eutectic mixture is reduced.

The preparing of the carnitine-salicylate (CA-SA) may include homogenizing the mixture using a homogenizer. At this time, a process of homogenizing the eutectic mixture until it becomes a colorless or light brown transparent phase may be included. Further purification may then be omitted.

According to the present invention, the components constituting the eutectic mixture are uniformly mixed under the above homogenization conditions, so that the skin improvement effect of the eutectic mixture, mild irritation to the skin, and excellent formulation stability may be realized.

When carnitine-salicylate (CA-SA) is completely dissolved, it maintains its liquid state and transparency even after a cooling process. The eutectic mixture prepared as described above may maintain a transparent liquid phase without phase separation even at a very low temperature, for example, a temperature less than 0 °C. In addition, the bond of the eutectic mixture may be maintained without being broken even when solidification and melting processes are repeated.

The preparing of the carnitine-salicylate (CA-SA) is performed at pH of 3.0 to 7.0. Carnitine-salicylate (CA-SA) is sufficiently soluble within the above acidity range and may maintain its efficacy.

In the preparing of the carnitine-salicylate (CA-SA), a eutectic mixture having a carnitine : salicylate molar ratio of 1 to 6:2, for example, 6:2, 5:2, 4:2, 3:2, 2:2, 1:2, 5.5:2, 4.5:2, 3.5:2, 2.5:2, or 1.5:2 may be used.

In the preparing of the cosmetic composition, 0.01 to 20 parts by weight, for example, 0.01 to 20 parts by weight, 0.01 to 1.2 parts by weight, 0.01 to 1.8 parts by weight, 0.01 to 2.5 parts by weight, 0.01 to 4.7 parts by weight, 0.1 to 7.3 parts by weight, 0.01 to 10 parts by weight, 1.2 to 20 parts by weight, or 1.8 to 10 parts by weight of carnitine-salicylate (CA-SA) may be added, based on 100 parts by weight of the total composition.

The preparation of carnitine-salicylate (CA-SA) according to the present invention is not limited by the types of carnitine and salicylate.

[Advantageous Effects]

The cosmetic composition according to the present invention includes carnitine-salicylate (CA-SA) as an active ingredient, so that it can exhibit acne relief and exfoliation efficacy as a skin improving material due to ion pairing of carnitine and salicylate in weakly acidic conditions, and at the same time, it can relieve inflammation and erythema as a skin soothing material, and further improve skin safety.

[Brief Description of Drawings]

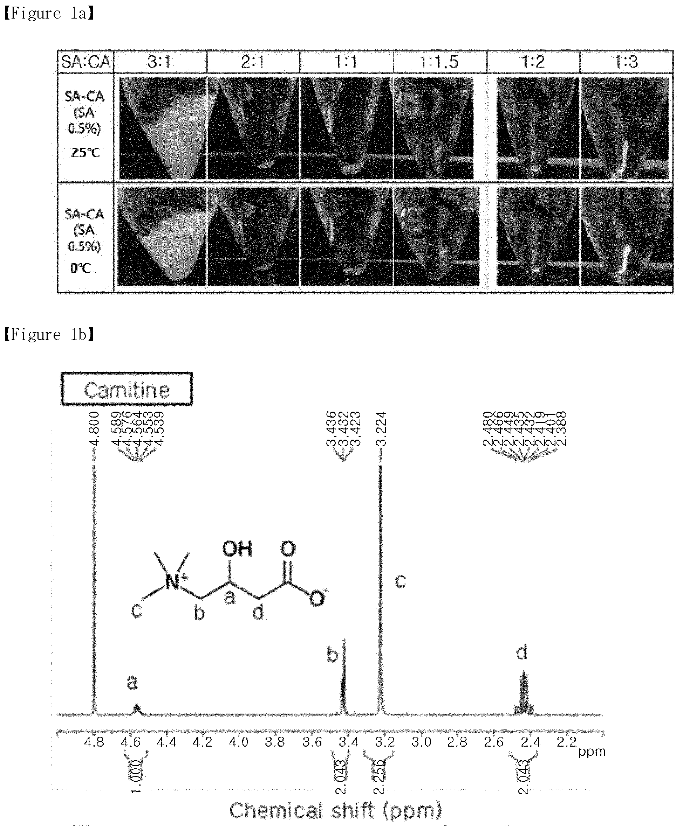

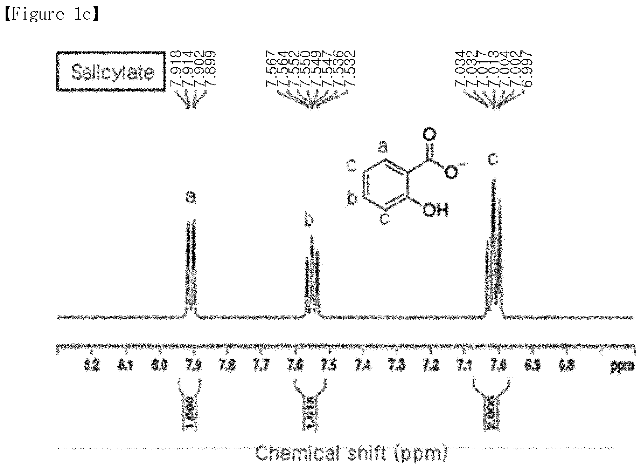

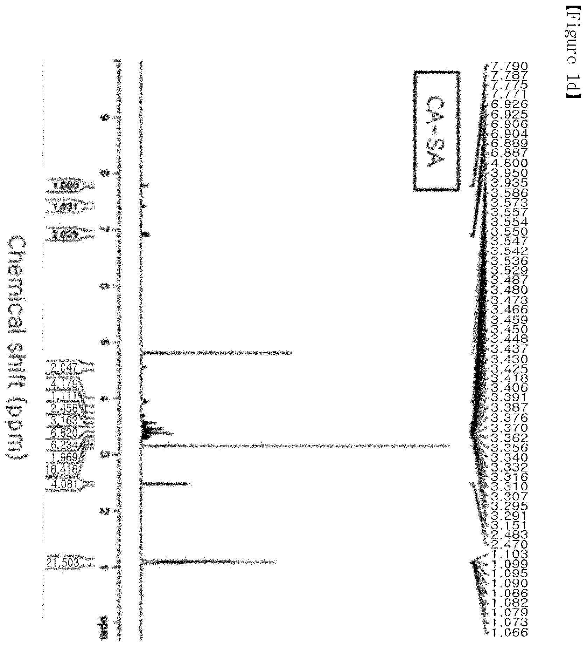

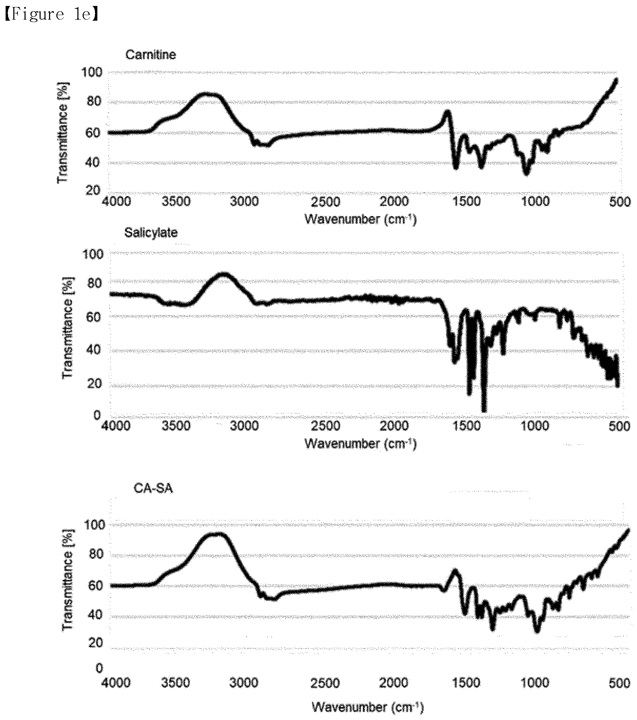

FIG. 1 is a macroscopic and chemical analysis result of carnitine-salicylate (CA-SA) according to the present invention. FIG. 1A is the result of confirming the macroscopic stability according to the molar ratio of carnitine-salicylate (CA-SA), FIG. 1B is an $^1$H NMR spectrum of carnitine, FIG. 1C is an $^1$H NMR spectrum of salicylate, FIG. 1D is an $^1$H NMR spectrum of carnitine-salicylate (CA-SA), and FIG. 1E is an IR spectrum of carnitine, salicylate, and carnitine-salicylate (CA-SA).

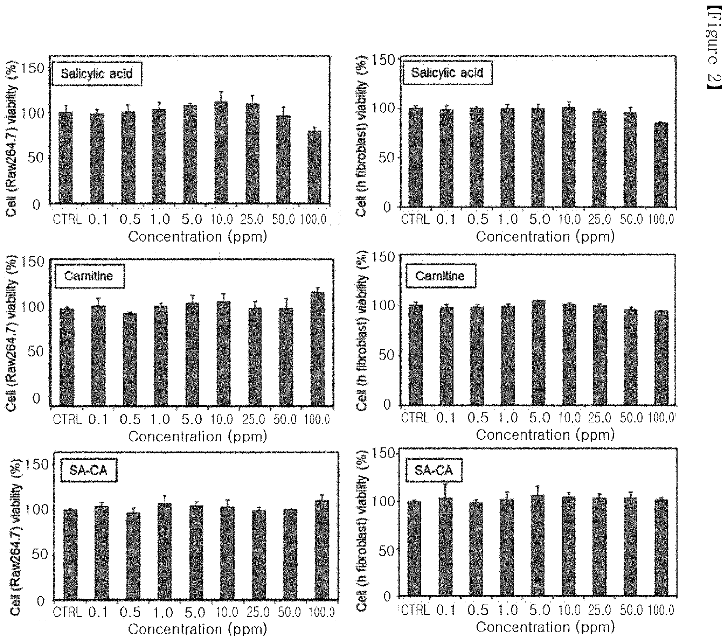

FIG. 2 is a result of confirming the *in vitro* cytotoxicity of carnitine, salicylate, and carnitine-salicylate (CA-SA).

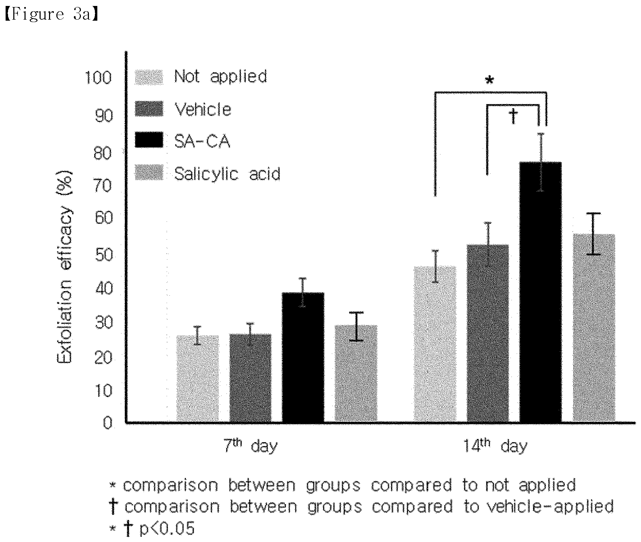

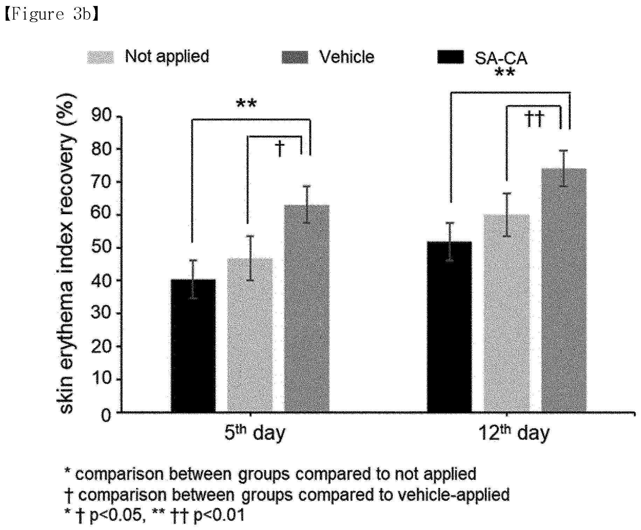

FIG. 3 is a result of confirming the *in vivo* efficacy of carnitine-salicylate (CA-SA) according to the present invention. FIG. 3A is the result of confirming the exfoliation efficacy of a cosmetic composition including carnitine-salicylate (CA-SA), and FIG. 3B is the result of confirming the erythema relief effect of a cosmetic composition including carnitine-salicylate (CA-SA).

[Best Mode for Implementation of the Invention]

The present invention relates to a cosmetic composition comprising carnitine-salicylate (CA-SA) as an active ingredient.

In the present invention, the carnitine is 4-trimethylamino-3-hydroxybutyric acid, and may include L-carnitine, DL-carnitine, hydrochlorides thereof, and derivatives thereof. Preferably, L-carnitine is used.

Carnitine has been reported as a hypoallergenic amino acid exfoliating agent that exhibits higher efficacy than $\alpha$-hydroxy acid (AHA) under weakly acidic and neutral conditions. In addition, since it has an ammonium group in its structure, it is advantageous for skin penetration and facilitates penetration into the stratum corneum.

An external skin preparation containing carnitine and exhibiting an acne improvement effect is disclosed (Korean Patent Publication No. 10-2010-0095447). However, the related art only uses carnitine as an additional additive as a ceramide synthesis promotor, and the mixing ratio thereof is different, and it is not included as a major component for the major effect. In addition, though the acne bacillus test and sensory evaluation test results are described for the acne improvement effect, objective results based on standard test methods for cytotoxicity, exfoliation, inflammation relief and erythema relief efficacy of external skin preparations are not presented.

In one embodiment of the present invention, as a result of preparing carnitine-salicylate (CA-SA) and conducting *in vitro* and *in vivo* efficacy tests, the material showed little cytotoxicity, so it may exhibit acne relief and exfoliation efficacy as a skin improvement material without irritating the skin, and at the same time the inflammation and erythema relief effect as a skin soothing material may be maximized. Salicylic acid, which is commonly used, has low solubility in water, so it is difficult to apply it in high concentrations to cosmetics, and application formulations are limited and may cause skin irritation. Therefore, it is recommended to use it in the form of salicylate, but this also has limitations in that skin permeability and efficacy are lowered when applied in weak acid systems.

A method of incorporating salicylic acid into a hydroxyalkylated cyclodextrin to improve its solubility in water and including it into a cosmetic composition at a high content is known (Korean Registered Patent No. 10-2307186), and it is also known that a cosmetic composition containing a certain salicylic acid derivative may inhibit tyrosinase activity to exhibit a skin whitening effect (Korean Patent Publication No. 10-2017-0076091). However, the related art suggests a cosmetic composition including salicylic acid as a derivative. In addition, only tyrosinase inhibition results and sensory evaluation test results are described for skin improvement effects, and objective results based on standard test methods regarding the cytotoxicity, acne relief, exfoliation, inflammation relief and erythema relief efficacy of external preparations have not been presented.

In one embodiment of the present invention, as a result of preparing carnitine-salicylate (CA-SA) and conducting *in vitro* and *in vivo* efficacy tests, the material showed little cytotoxicity, so it may exhibit acne relief and exfoliation efficacy as a skin improvement material without irritating the skin, and at the same time the inflammation and erythema relief effect as a skin soothing material may be maximized.

In the present invention, a eutectic mixture may be prepared and used in the form of carnitine-salicylate (CA-SA) in which salicylic acid is ion-paired with carnitine, instead of using salicylic acid in the form of a salt. By including carnitine-salicylate (CA-SA), the cosmetic composition according to the present invention may be prepared under weakly acidic conditions, and may exhibit more improved effects than the original efficacy of each component. Specifically, the skin improvement effect can be improved by 120% or more, or 140% or more, or 160% or more compared to a simple mixture. In addition, the cosmetic composition according to the present invention may improve the stability of the composition by including carnitine-salicylate (CA-SA). In addition, since the material has little cytotoxicity, it shows acne relief and exfoliation efficacy as a skin improvement material without irritating the skin, and at the same time, maximizes an inflammation and erythema relief effect as a skin soothing material.

The eutectic mixture means a mixture of two or more solid or liquid substances, and means that two components with high melting points are mixed and form a complex by dipole-dipole attraction between polar molecules, dipole-induced dipole attraction, hydrogen bonding between molecules, or van der Waals interactions. In other words, the bonding of the eutectic mixture refers to the bonding between molecules (formation of eutectic mixture) in a state in which the unique molecular structure of each individual is maintained based on hydrogen bonding and bonding between dipoles by the partial charge of molecules.

In addition, the eutectic mixture may be formed by combining polar compounds. The polar compound may be a polar molecule or a charged molecule. In addition, there needs to be a difference in polarity between the polar compounds, and the same compounds cannot form a eutectic mixture.

In the eutectic mixture, the ratio between molecules required for the eutectic mixture may be determined according to the charge of the molecules of the components constituting the eutectic mixture, the type and number of functional groups of the molecules, and the polarity of the molecules or functional groups. In addition, a ratio between molecules required for the eutectic mixture may be determined according to the size or similarity of molecules.

In the present invention, for carnitine-salicylate (CA-SA), the ratio between molecules required for the eutectic mixture was determined according to the charge and functional group of carnitine and salicylate molecules, and the polarity of the molecule or functional group. The carnitine-salicylate (CA-SA) may be mixed in a carnitine : salicylate molar ratio of 1 to 6:2, for example, 6:2, 5:2, 4:2, 3:2, 2:2, 1:2, 5.5:2, 4.5:2, 3.5:2, 2.5:2, or 1.5:2. It was confirmed, in Experimental Example 1-1, that carnitine-salicylate (CA-SA) was stable within the above molar ratio range.

Carnitine and salicylate can form a eutectic mixture due to bonding between dipoles by the partial charge of a specific functional group, and may form a eutectic mixture at a molar ratio within the above range through their chemical structures.

When the molar ratio is out of the above range, carnitine has degraded low-temperature stability, and salicylate may be precipitated at a low temperature due to remaining salicylate molecules that cannot ion-pair with carnitine, that is, as their water solubility is exceeded.

In the present invention, the carnitine-salicylate (CA-SA) may preferably be in a transparent phase without precipitate when left at a low temperature. More specifically, no precipitate may be formed even when left at 0 °C for 3 weeks, 4 weeks, 5 weeks, or 6 weeks or more.

The cosmetic composition of the present invention may be in the form of a general emulsified formulation and a solubilized formulation. For example, the cosmetic composition may have formulations of a toner such as a skin softening toner or a nutritional skin toner, a lotion such as a facial lotion or a body lotion, a cream such as a nourishing cream, a moisturizing cream, or an eye cream, an essence, a cosmetic ointment, a balm, a spray, a gel, a pack, a sunscreen, a makeup base, a liquid-, solid-, or spray-type foundation, a powder, a makeup remover such as a cleansing cream, a cleansing lotion, or a cleansing oil, a cleanser such as cleansing foam, soap, body wash, and the like.

In addition, the cosmetic may contain, in addition to the composition of the present invention, adjuvants commonly used in cosmetology, such as fatty materials, organic solvents, solubilizing agents, thickening agents, gelling agents, softening agents, antioxidants, suspending agents, stabilizers, foaming agents, fragrances, surfactants, water, ionic or nonionic emulsifiers, fillers, sequestering and chelating agents, preservatives, vitamins, blocking agents, wetting agents, essential oils, dyes, pigments, hydrophilic or lipophilic active agents, lipid vesicles, or any other ingredient commonly used in cosmetics.

The cosmetic formulation may include a relatively high concentration of the composition in a wash-off type cosmetic such as a make-up remover, cleanser, and the like in which active ingredients remain on the skin for a short period of time. On the other hand, in leave-on type cosmetics such as toners, lotions, creams, essence, and the like in which active ingredients remain on the skin for a long time, the cosmetics may also include a low concentration of the composition compared to wash-off type cosmetics. Although not limited thereto, in one embodiment of the present invention, the composition of the present invention may include 0.01 to 20 parts by weight, for example, 0.01 to 20 parts by weight, 0.01 to 1.2 parts by weight, 0.01 to 1.8 parts by weight, 0.01 to 2.5 parts by weight, 0.01 to 4.7 parts by weight, 0.1 to 7.3 parts by weight, 0.01 to 10 parts by weight, 1.2 to 20 parts by weight, or 1.8 to 10 parts by weight of carnitine-salicylate (CA-SA), based on the total weight of the composition.

When the carnitine salicylate (CA-SA) according to the present invention is included in an amount less than 0.01 part by weight based on the total weight of the composition, sufficient acne relief, exfoliation promotion, inflammation relief and erythema relief effects cannot be expected, and when it is included in an amount of more than 20 parts by weight, there may be unwanted reactions such as allergies or problems with skin safety.

Each of the above components included in the cosmetic composition according to the present invention may be included in the cosmetic composition of the present invention within a range that does not exceed the maximum amount prescribed in each country's cosmetic safety standards.

In addition, the present invention provides a method of preparing a cosmetic composition comprising carnitine-salicylate (CA-SA).

In another aspect, the present invention provides a method for alleviating inflammation and erythema for skin soothing while exhibiting acne relief and exfoliation effects for skin improvement, including applying a cosmetic composition comprising carnitine-salicylate (CA-SA) as an active ingredient to the skin.

In another aspect, the present invention provides a method for alleviating inflammation and erythema for skin soothing while exhibiting acne relief and exfoliation effects for skin improvement, including administering a cosmetic composition comprising carnitine-salicylate (CA-SA) as an active ingredient to a subject.

In another aspect, the present invention provides a use of a composition comprising carnitine-salicylate (CA-SA) as an active ingredient, for the preparation of cosmetics for alleviating inflammation and erythema for skin soothing while exhibiting acne relief and exfoliation effects for skin improvement

Hereinafter, the configuration of the preparation method of the present invention will be described in detail.

The preparation method of the present invention may comprise preparing a mixture of carnitine and salicylic acid; stirring the mixture of the carnitine and salicylic acid at 50 °C to 70 °C to prepare carnitine-salicylate (CA-SA); and adding the carnitine-salicylate (CA-SA) to prepare a cosmetic composition.

The preparing of the mixture of carnitine and salicylic acid may include adding purified water.

In the preparing of the carnitine-salicylate (CA-SA), when the temperature is less than 50 °C, eutectic bonding may not be sufficiently performed, and when the temperature exceeds 70 °C, the eutectic bonding may be broken and the effect of the eutectic mixture is reduced.

The preparing of the carnitine-salicylate (CA-SA) may include homogenizing the mixture using a homogenizer. At this time, a process of homogenizing the eutectic mixture until it becomes a colorless or light brown transparent phase may be included. Further purification may then be omitted.

According to the present invention, the components constituting the eutectic mixture are uniformly mixed under the above homogenization conditions, so that the skin improvement effect of the eutectic mixture, mild irritation to the skin, and excellent formulation stability may be realized.

When carnitine-salicylate (CA-SA) is completely dissolved, it maintains its liquid state and transparency even after a cooling process. The eutectic mixture prepared as described above may maintain a transparent liquid phase without phase separation even at a very low temperature, for example, a temperature less than 0 °C. In addition, the bond of the eutectic mixture may be maintained without being broken even when solidification and melting processes are repeated.

The preparing of the carnitine-salicylate (CA-SA) is performed at pH of 3.0 to 7.0. Carnitine-salicylate (CA-SA) is sufficiently soluble within the above acidity range and may maintain its efficacy.

In the preparing of the carnitine-salicylate (CA-SA), a eutectic mixture having a carnitine : salicylate molar ratio of 1 to 6:2, for example, 6:2, 5:2, 4:2, 3:2, 2:2, 1:2, 5.5:2, 4.5:2, 3.5:2, 2.5:2, or 1.5:2 may be used.

In the preparing of the cosmetic composition, 0.01 to 20 parts by weight, for example, 0.01 to 20 parts by weight, 0.01 to 1.2 parts by weight, 0.01 to 1.8 parts by weight, 0.01 to 2.5 parts by weight, 0.01 to 4.7 parts by weight, 0.1 to 7.3 parts by weight, 0.01 to 10 parts by weight, 1.2 to 20 parts by weight, or 1.8 to 10 parts by weight of carnitine-salicylate (CA-SA) may be added, based on 100 parts by weight of the total composition.

The preparation of carnitine-salicylate (CA-SA) according to the present invention is not limited by the types of carnitine and salicylate.

[Modes of the Invention]

Hereinafter, the present invention will be described in detail through Examples. The following examples merely illustrate the present invention, but the scope of the present invention is not limited by the following examples. The examples are merely provided to complete the disclosure of the present invention and to fully convey the scope of the invention to those skilled in the art, and the present invention is only defined by the scope of the claims.

Preparation Example 1: Preparation of carnitine-salicylate (CA-SA)

Carnitine and salicylate were mixed at a molar ratio of 1 to 6: 2, where a small amount of purified water was added and stirred at a temperature between 50 to 70 °C until the mixture became homogeneous and transparent. At this time, each preparation was made using a solution with a final salicylate content of 0.5% and a pH of 5.5, which is a weak acid system. Thereafter, no further purification was performed. A eutectic mixture solution in which ion pairing was formed stably had no precipitate.

**Experimental Example 1: Analysis of carnitine-salicylate (CA-SA)**

<Experimental Example 1-1> Evaluation of solubility and stability of carnitine-salicylate (CA-SA)

In order to confirm the stability of carnitine-salicylate (CA-SA), the carnitine-salicylate (CA-SA) prepared in the above Preparation Example was left at 25 °C and 0 °C for 4 weeks and macroscopic changes in properties were observed for evaluation. After 4 weeks, phase separation and precipitation were observed with the naked eye, and the results are shown in Table 1 below and FIG. 1A.

[Table 1]

|  | CA: SA Molar ratio | 25 °C | 0 °C |
|---|---|---|---|
| Example 1 | 3:1 | Stable | Stable |
| Example 2 | 2:1 | Stable | Stable |
| Example 3 | 1:1 | Stable | Stable |
| Example 4 | 1:2 | Stable | Stable |
| Comparative Example 1 | 1:3 | Precipitation occurs after 3 days | Precipitation occurs after 1 day |
| CA: Carnitine SA: Salicylate | | | |

As shown in Table 1 and FIG. 1A, through the results of Examples 1 to 4, it was confirmed that carnitine salicylate (CA-SA) was stable when it had a molar ratio of carnitine: salicylate = 1 to 6:2. In the ratio of carnitine: salicylate = 1: 3 of Comparative Example 1, as salicylate seems to precipitate at a low temperature as the content of the remaining salicylate in the water exceeds its water solubility.

Through the above results, as carnitine-salicylate (CA-SA) was stable at room temperature and low temperature, it was concluded that it could be applied to a cosmetic composition.

**<Experimental Example 1-2> $^1$H-NMR analysis**

In order to perform comparative analysis of the structures of carnitine, salicylate, and carnitine-salicylate (CA-SA), $^1$H-NMR spectra were measured at 35 °C using AvanceIII-500 (NMR spectrometer, Bruker Instruments, USA) under 500 MHz conditions. Sample preparation for $^1$H-NMR measurement was performed using $D_2O$. Measurements were performed under conditions of a 90° pulse width of 12.2 $\mu s$; a relaxation delay of 4s; and 100 scans. Tetramethylsilane was used as a standard material. At this time, structural analysis of carnitine-salicylate (CA-SA) was performed using Example 2 of Experimental Example 1-1. The results are shown in FIGS. 1B, 1C, and 1D.
As shown in FIGS. 1B, 1C, and 1D, $^1$H-NMR spectrum results of individual components constituting carnitine-salicylate (CA-SA) and the $^1$H-NMR spectrum results of carnitine-salicylate (CA-SA) are shown. A decrease in the number of split peaks after the formation of carnitine-salicylate (CA-SA) was confirmed in the peak of d in which carnitine spin-spin splitting occurred before and after formation of carnitine-salicylate (CA-SA). Corresponding changes were also confirmed in salicylate, as all peaks a, b, and c were shifted upward. In particular, the upward shift value was the largest in a, and through this, it was inferred that the functional group participating in the binding of carnitine-salicylate (CA-SA), in salicylate, was a carboxyl group. Through the change in the number of hydrogens around the d hydrogen of carnitine and the occurrence of intermolecular bonding of the salicylate carboxyl group, it was inferred that carnitine and salicylate form a hydrogen bond in which the protons are shared.
From the above, it was confirmed that carnitine and salicylate actually become ion-pairs.

**<Experimental Example 1-3> FT-IR analysis**

In order to confirm which functional groups of carnitine and salicylate bind together to form carnitine-salicylate (CA-SA), FT-IR spectrum analysis was performed. The FT-IR spectrum was measured at 2 $cm^1$ intervals in the range of 3600 to 500 $cm^1$ using a Vertex70, Hyperion 2000 (FTIR spectrophotometer, Bruker Instruments) model. Thereafter, the spectra were analyzed by deconvolution using Opus 5.5 software (Bruker Instruments). At this time, structural analysis of carnitine-salicylate (CA-SA) was performed using Example 2 of Experimental Example 1-1. The results are shown in FIG. 1E.
As shown in FIG. 1E, first, in the IR spectrum of L-carnitine, an enhancement of the IR peak is observed due to vibrations caused by -COOH and -OH groups. The positions of these main peaks are 1396-1385 $cm^{-1}$ (COOH), 1245 $cm^{-1}$ (CC), 1137-1195 $cm^{-1}$ (CCO, COH), 913 $cm^{-1}$ (C-COOH), and 625-634 $cm^{-1}$ (COH), and the band due to an ammonium group also appears characteristically. The peaks of this group (C-$N^+$ ($CH_3)_3$) are characterized by three bands at 970, 944 and 772 $cm^{-1}$. In the case of salicylate, the characteristic vibration peaks at 3233 $cm^{-1}$ and 999-2831 $cm^{-1}$ due to OH and CH, respectively, and C=O ($COO^-$) peaks at 1652-1670 $cm^{-1}$ and 1386 $cm^{-1}$ were observed. In addition, a peak due to C=C (phenol group) was observed at 1558-1610 $cm^{-1}$, and characteristic peaks were observed at 1324 $cm^{-1}$ (OH; phenol group), 1296 $cm^{-1}$ ($COO^-$ (C-O)), and 1156-1248 $cm^{-1}$ (C-OH; phenol group). When checking the change of these peaks after the formation of carnitine-salicylate (CA-SA), changes in the spectral form due to intermolecular bonding at 1137-1195 $cm^{-1}$ (CCO, COH) and 625-634 $cm^{-1}$ (COH) peaks of carnitine and the 1652-1670 $cm^{-1}$ C=O ($COO^-$) position of salicylate could be observed. Through this, as in the results of $^1$H-NMR analysis, it was concluded that carnitine-salicylate (CA-SA) forms a hydrogen bond in which the OH and $COO^-$ groups of carnitine and salicylate share protons.
From the above, it was confirmed that carnitine and salicylate actually become ion-pairs.

**Experimental Example 2:** Evaluation of *in vitro* exfoliation, cytotoxicity, and anti-inflammatory efficacy of carnitine-salicylate (CA-SA)

**<Experimental Example 2-1>** Confirmation of *in vitro* exfoliation efficacy of carnitine-salicylate (CA-SA)

In order to compare the exfoliation efficacy of carnitine (CA), salicylate (SA), a simple mixture of carnitine and salicylate (SA + CA, MIX) and carnitine-salicylate (SA-CA), the relative dead skin cell control efficacy was evaluated *in vitro* using pig back skin (Apures, Republic of Korea) at pH 5.5, which is a weakly acidic condition that does not cause skin irritation. The pig back skin surface was washed with phosphate buffer saline (PBS) and then cut to a certain size. Thereafter, the stratum corneum of the pig skin was exposed and placed on a 96-well plate, 100 $\mu l$ of each sample was applied, and the 96-well plate was left in a constant temperature and humidity chamber for 16 hours. Thereafter, the supernatant of each sample was mixed well with a trypan blue solution in the 96-well plate to measure the number of detached keratinocytes. In order to verify the suitability of the relative dead skin cell control efficacy evaluation method used in this study, the following method was used. DL-gluconolactone at pH 4.0 and 6.0 were used as a positive control.

After treating the pig skin with the sample by the method described above, the number of detached keratinocytes was counted, and the number of detached keratinocytes in PHA (DL-gluconolactone) at pH 4.0 was set to 100% to evaluate the relative exfoliation efficacy. All evaluations were made at n = 5 to secure reliability through repeated results. The exfoliation efficacy was compared and evaluated using the average and standard deviation of the number of detached keratinocytes, and the results are shown in Table 2.

[Table 2]

|  | Exfoliation rate (%) |
|---|---|
| Gloconolactone 10%, pH 4 | 100.0 |
| Gloconolactone 10%, pH 6 | 32.0 |
| CA2% | 55.2 |
| SA 2% | 19.2 |
| SA-CA 2% | 91.8 |
| SA 1% + CA 1% | 56.5 |

As shown in Table 2, when compared to the efficacy of 10.0% gluconolactone at pH 4.0, wherein the efficacy was assumed as 100% and the other results were obtained relatively to this result, carnitine-salicylate (CA-SA) exhibited an exfoliation efficacy of 91.8%. On the other hand, the same amount of salicylate showed an exfoliation efficacy of 19.2%, carnitine showed an exfoliation efficacy of 55.2%, and a simple mixture of carnitine and salicylate (CA+SA, MIX) showed an exfoliation efficacy of 56.5%. Through the above results, it was concluded that carnitine-salicylate (CA-SA) shows remarkable exfoliation effect even in weak acid systems, which causes little irritation to the skin .

**<Experimental Example 2-2>** Confirmation of *in vitro* cytotoxicity of carnitine-salicylate (CA-SA)

The cytotoxicity of carnitine-salicylate (CA-SA) was evaluated by 3-(4,5-dimethythiazol-2-yl)-2,5-diphenyl tetrazolium bromide (MTT) assay. A Raw 264.7 cell line and fibroblasts obtained from human skin tissue were seeded at a concentration of 1 to 2 $\times$ 10$^5$ cells/ml in a 24-well plate using a DMEM medium supplemented with 10% FBS and incubated in an incubator at 5% $CO_2$ for 24 hours. After removing the medium and adding each sample to a medium without FBS and incubating for 24 hours, the medium was removed, an MTT solution was added at a concentration of 1 $\mu$g/ml, and reacted at 37 °C for 3 hours. The unreacted MTT solution was removed, and 100 $\mu$l of DMSO was added to dissolve the formed reaction product, and then absorbance was measured at 540 nm to confirm the cytotoxicity.

The cytotoxicity of salicylate, carnitine, and carnitine-salicylate (CA-SA) was evaluated by treating Raw 264.7 cells, which are rat macrophages, and human fibroblasts with the material. As a Raw 264.7 cell is a representative cell used to measure NO inhibitory efficacy in anti-inflammatory efficacy evaluations, its cytotoxicity was evaluated, and cytotoxicity with respect to fibroblasts was evaluated in order to confirm the toxicity that may be induced when the material is included in cosmetics and be repeatedly applied to the skin. At this time, the carnitine-salicylate (CA-SA) of Example 2 of Experimental Example 1-1 was used and the evaluation was performed based on a content of salicylic acid in the eutectic compound rather than a content of carnitine-salicylate (CA-SA). The results are shown in FIG. 2.

As shown in FIG. 2, it was confirmed that when Raw 264.7 cells were treated with salicylate, the cell viability was 96.16% in 50 ppm-treated cells and 79.74% in 100 ppm-treated cells, and when fibroblasts were treated with salicylate, the cell viability was 94.83% in 50 ppm-treated cells, and 85.36% in 100 ppm-treated cells. This is a result that may induce cytotoxicity when salicylate is treated at 50 ppm or more at the cellular level. However, in the case of carnitine and carnitine-salicylate (CA-SA), the cell viability was 95% or more in the entire concentration range treated up to 100 ppm and showed no cytotoxicity.

Through the above results, since the ion paired carnitine-salicylate does not show the cytotoxicity inherent in each component, it can be concluded that carnitine and salicylate can be safely used in the human body as it has little effect on cell activity.

**<Experimental Example 2-3>** Confirmation of *in vitro* anti-inflammatory efficacy of carnitine-salicylate (CA-SA)

The anti-inflammatory effect of carnitine-salicylate (CA-SA) was confirmed experimentally. Based on the results of Experimental Example 2-2, the anti-inflammatory effect of salicylate, carnitine, and carnitine-salicylate (CA-SA) at 10 ppm, the concentration which all materials did not exhibit cytotoxicity, were evaluated by their NO inhibitory effects. 500 ng/ml of lipopolysaccharide (LPS), which is an inflammation-inducing material, was treated to confirm the NO inhibition rate, where 100 mM of NG-monomethyl-L-arginine acetate (L-NMMA), a nitric oxide synthase (NOS) inhibitor, was treated in the positive control.

In order to evaluate the efficacy of relieving inflammation caused by external stimuli *in vitro*, an experiment was performed by the GRIESS method using Raw 264.7 cells (ATCC number: CRL-2788), which are rat macrophage cell line, to evaluate the inhibition of NO production after treatment with lipopolysaccharide (LPS, Sigma Aldrich, USA). Raw 264.7 cells pre-cultured in a growth medium (DMEM with 10% FBS, Welgene, USA) were added to a 24-well tissue culture plate at a concentration of 1 to $2 \times 10^5$ cells/ml, and cultured in an incubator for one day in a 5% $CO_2$ environment. After removing the medium and performing starvation with a serum-free medium for 12 hours, the test material was treated at 0.1 to 100 ppm for 30 minutes, and then LPS was added at a concentration of 500 ng/ml and the cells were further cultured for 18 hours. After culturing, the supernatant was taken and transferred to a 96-well plate, GRIESS reagent was added and reacted at room temperature for 15 minutes, and absorbance at 540 nm was measured using an ELISA reader. Carnitine-salicylate (CA-SA) of Example 2 of Experimental Example 1-1 was used. The results are shown in Table 3.

[Table 3]

|  | NO inhibition rate |
|---|---|
| Negative control | 0% |
| L-MMMA (100 mM) | 62.2% |
| SA 10 ppm | 16.4% |
| CA 10 ppm | 0.0% |
| SA-CA 10 ppm | 60.0% |
| SA 10 ppm + CA 10 ppm | 20.4% |

As shown in Table 3, salicylate showed an NO inhibition efficacy of 16.4%, carnitine showed an NO inhibition efficacy of 0.0%, a simple mixture of carnitine and salicylate (SA + CA) showed an NO inhibition efficacy of 20.4%, and carnitine-salicylate (CA-SA) showed an NO inhibition efficacy of 60.0%. Through the above results, it can be confirmed that the carnitine salicylate (CA-SA) according to the present invention showed better anti-inflammatory efficacy than the anti-inflammatory effect of each component through ion pairing, and it can be confirmed that it is an advanced material capable to show a skin soothing effect against irritation that may be caused by the unique exfoliation effect of each component.

**<Experimental Example 2-4>** Confirmation of *in vitro Propionibacterium acnes* bactericidal efficacy of carnitine-salicylate (CA-SA)

The *Propionibacterium acnes* bactericidal efficacy of carnitine-salicylate (CA-SA) was confirmed through a time-killing assay. First, the sample was inoculated with bacteria so that the concentration was $10^6$ CFU/ML. When 20 minutes, 1 hour, 3 hours, or 6 hours had elapsed after inoculation, 1 ml was sampled at each time, diluted in a D/E solution, and then antiseptic ability was removed. Each of the following samples was diluted at an appropriate dilution factor and plated on an mLNA medium. After culturing each sample for 48 hours or more in a 30 °C incubator, the number of bacteria in the plate was confirmed. Samples were prepared as shown in Table 4 below, and the results are shown in Table 5 below.

[Table 4]

|  | Example 5 | Comparative Example 2 | Comparative Example 3 | Comparative Example 4 |
|---|---|---|---|---|
| Composition | CA-SA 0.5% | SA 0.5% | CA 0.5% | SA 0.5% |
| pH | 5.5 | 5.5 | 6.0 | 2.7 |
| **SA: salicylate, CA: carnitine, CA-SA: carnitine-salicylate** | | | | |

[Table 5]

| Elapsed time | Example 5 | Comparative Example 2 | Comparative Example 3 | Comparative Example 4 |
|---|---|---|---|---|
| 1h | <100 | $5.02 \times 10^5$ | $4.96 \times 10^5$ | <100 |
| 3h | <100 | $1.75 \times 10^5$ | $4.96 \times 10^5$ | <100 |
| 6h | <100 | $2.00 \times 10^3$ | $3.44 \times 10^5$ | <100 |
| 24h | <100 | <100 | $2.04 \times 10^5$ | <100 |

It was confirmed that the acne bacteria sterilization rate of Example 5 was significantly higher than the acne bacteria sterilization rate of Comparative Example 2 and Comparative Example 3, which are single components in weakly acidic conditions, and despite being weakly acidic, the efficacy is similar to that of Comparative Example 4, which has the lowest pH. Therefore, it can be concluded that the carnitine-salicylate (CA-SA) according to the present invention can improve acne-prone skin without irritation by sufficiently removing acne bacteria even in weak acid systems.

**Experimental Example 3:** Evaluation of *in vivo* exfoliation and erythema inhibition efficacy of carnitine-salicylate (CA-SA)

**<Experimental Example 3-1>** Confirmation of *in vivo* exfoliation efficacy of carnitine-salicylate (CA-SA)

In order to evaluate the exfoliation efficacy of carnitine salicylate (CA-SA), 6 random volunteers (age: 25 to 35 years old) for a clinical trial participated in the experiment. 5.0% DHA was applied to the subject's forearm and maintained for 24 hours to dye the keratin. The stained area was evaluated using a color difference meter (CR-400, Konica Minolta, Japan), and 4 areas with the same staining level were selected. An aqueous solution of carnitine-salicylate (CA-SA) (Example 6) and a base including no CA-SA (Comparative Example 5) were applied to the same area twice a day (morning and evening) for 14 days. One area is was not applied for comparison. All areas were maintained under the same conditions, and no additional physical or chemical exfoliation was performed during the evaluation period. After 7 days and 14 days, the exfoliation efficacy was evaluated by the change in brightness of the stained areas using the color difference meter. The exfoliation efficacy was evaluated as follows.

$$Exfoliation_t(\%) = \frac{(colorimeter\ value_0 - colorimeter\ value_t)}{(colorimeter\ value_0 - colorimeter\ value_{-1})} \times 100$$

*t: measurement time point, -1: time point before keratin staining, 0: time point after keratin staining*

Statistical significance was analyzed using a paired t-test. The base for evaluation was prepared so as not to include a material that could affect exfoliation, inflammation induction and relief, and the results are shown in FIG. 3A.

[Table 6]

| Component name | Example 6 | Comparative Example 5 |
|---|---|---|
| Deionized water | To 100 | To 100 |
| Carnitine-salicylate (SA-CA) | 5.00 | - |
| 1,2-Hexanediol | 2.00 | 2.00 |
| Glycerin | 8.00 | 8.00 |
| 1,3-Butylene glycol | 3.00 | 3.00 |
| Cyclopentasiloxane | 7.00 | 7.00 |
| Dimethicone | 4.50 | 4.50 |
| Polysorbate 60 (Tween 60) | 1.50 | 1.50 |
| Acrylates/C10-30 alkylacrylate crosspolymer | 0.35 | 0.35 |
| Tromethamine | 0.35 | 0.35 |
| Total | 100 | 100 |

As shown in FIG. 3A, in the case of a non-application group and a base formulation application group (Comparative Example 5), the improvement rates were 4.65% and 10.41% on day 7, and 43.00% and 47.30% on day 14, respectively, and in the case of a carnitine salicylate (CA-SA) application group (Example 6), the improvement rates were 49.16% and 65.49%, thus it was confirmed that when carnitine salicylate (CA-SA) was applied, the exfoliation effect was improved by 38.46% on day 14 compared to the base application group.

Through the above results, it can be concluded that, by including the carnitine-salicylate (CA-SA) according to the present invention in a cosmetic composition, an exfoliation effect can be exhibited when applied to the human body.

<3-2> Confirmation of *in vivo* erythema inhibitory efficacy of carnitine-salicylate (CA-SA)

An experiment to confirm the erythema inhibitory efficacy was performed using Example 6 of Experimental Example 3-1. In order to evaluate the erythema inhibitory effect, the erythema index of 9 random volunteers (age: 25 to 35 years old) was evaluated, where the evaluation was performed before erythema induction, after erythema induction, 5 days and 12 days after product use. After inducing skin barrier damage and erythema by applying a 1% SDS diluted solution to the forearm for 24 hours, an aqueous solution of carnitine-salicylate (SA-CA) (0.5% salicylate, pH 5.5) and a base including no CA-SA were repeatedly applied to a designated area twice daily (morning and evening) for 12 days. After 5 days and 12 days, the erythema index was measured using Mexameter (MX 18, Courage+Khazaka electronic GmbH, Germany), and an erythema index recovery rate was evaluated as follows.

$$Recovery_t(\%) = \frac{(Erythema\ index_0 - Erythema\ index_t)}{(Erythema\ index_0 - Erythema\ index_{-1})} \times 100$$

*(t: product use day, -1: before erythema induction, 0: after erythema induction)*

Statistical significance was analyzed using a paired t-test. The results are shown in FIG. 3B.

As shown in FIG. 3B, after 5 days and 12 days, the unapplied subjects showed recovery rates of 40.32% and 51.82%, and subjects to whom the base formulation was applied showed recovery rates of 46.76% and 59.86%, respectively, while subjects to whom carnitine salicylate (CA-SA) was applied (Example 6) showed recovery rates of 63.08% after 5 days and 74.03% after 12 days, that is, subjects to whom CA-Sa was applied showed a 34.90% higher erythema recovery rate after 5 days and a 23.67% higher erythema recovery rate after 12 days compared to the subject to whom the base formulation was applied.

Through the above results, it can be concluded that by including the carnitine-salicylate (CA-SA) according to the present invention in a cosmetic composition, inflammation relief and erythema relief efficacy can be exhibited at the same time when applied to the human body.

**Experimental Example 4:** Sensory evaluation of cosmetic composition including carnitine-salicylate (CA-SA)

A sensory evaluation test was performed using Example 6 of Experimental Example 3-1.

In order to evaluate the sensory efficacy regarding keratin and skin soothing by applying a cosmetic composition including carnitine salicylate (CA-SA), evaluation was performed targeting volunteers with sensitive skin who used cosmetics every day but frequently complained of skin irritation and worry a lot about skin troubles. All volunteers used the cosmetic composition including carnitine-salicylate (CA-SA) according to the present invention in a first step after washing their face while using the cosmetics they normally use. Evaluation was performed using a 5-point scale (5 points: very much improved, 4 points: felt an improvement, 3 points: did not feel a change, 2 points: felt worse, and 1 point: felt very worse) and the evaluation results are shown in Table 7 below.

[Table 7]

| Test items | Average score |
|---|---|
| Soft feeling without irritation | 4.32 |
| Skin erythema removal | 3.50 |
| Skin soothing immediately after use | 3.67 |
| Rough skin feeling removal | 3.61 |
| Overall product preference | 4.12 |

As the above results, after periodically and repeatedly applying the cosmetic composition including carnitine-salicylate (CA-SA, Example 6), it was evaluated that skin irritation and redness were improved and skin texture was improved. The softening of the skin texture appears to be due to continuous exfoliation. From the above results, it can be concluded that by including carnitine-salicylate (CA-SA) in the cosmetic composition, daily exfoliation is possible with mild irritation, and relief of inflammation caused by stimulants can also be experienced.

[Industrial Applicability]

The cosmetic composition according to the present invention includes carnitine-salicylate (CA-SA) as an active ingredient, so that it can exhibit acne relief and exfoliation efficacy as a skin improving material due to ion pairing of carnitine and salicylate in weakly acidic conditions, and at the same time, it can relieve inflammation and erythema as a skin soothing material, and further improve skin safety.

**Claims**

1. A cosmetic composition comprising carnitine-salicylate (CA-SA) as an active ingredient.

2. The cosmetic composition of claim 1, wherein the cosmetic composition has a pH of 4.0 to 6.5.

3. The cosmetic composition of claim 1, wherein the carnitine-salicylate (CA-SA) is included in an amount of 0.01 to 20 parts by weight based on 100 parts by weight of the total composition.

4. The cosmetic composition of claim 1, wherein the carnitine-salicylate (CA-SA) is a eutectic mixture of carnitine and salicylate in a carnitine: salicylate molar ratio of 1 to 6: 2.

5. The cosmetic composition of claim 1, wherein the carnitine-salicylate (CA-SA) has no precipitate when left at 0 °C for 4 weeks.

6. The cosmetic composition of claim 1, wherein the cosmetic composition is for skin improvement.

7. The cosmetic composition of claim 6, wherein the skin improvement is for acne relief.

8. The cosmetic composition of claim 6, wherein the skin improvement is for exfoliation promotion.

9. The cosmetic composition of claim 1, wherein the cosmetic composition is for skin soothing.

10. The cosmetic composition of claim 9, wherein the skin soothing is for inflammation relief.

11. The cosmetic composition of claim 9, wherein the skin soothing is for erythema relief.

12. A method of preparing a cosmetic composition, comprising:

    preparing a mixture of carnitine and salicylic acid;
    stirring the mixture at 50 °C to 70 °C to prepare carnitine-salicylate (CA-SA); and
    adding the carnitine-salicylate (CA-SA) to prepare a cosmetic composition.

13. The method of claim 12, wherein the preparing of the mixture of carnitine and salicylic acid includes adding purified water.

14. The method of claim 12, wherein the preparing of the carnitine-salicylate (CA-SA) includes homogenizing using a homogenizer until the carnitine-salicylate (CA-SA) becomes a colorless or light brown transparent phase.

15. The method of claim 12, wherein the preparing of the cosmetic composition includes adding 0.01 to 20 parts by weight of carnitine-salicylate (CA-SA) based on 100 parts by weight of the total composition.

【Figure 1a】

| SA:CA | 3:1 | 2:1 | 1:1 | 1:1.5 | 1:2 | 1:3 |
|---|---|---|---|---|---|---|
| SA-CA (SA 0.5%) 25°C | | | | | | |
| SA-CA (SA 0.5%) 0°C | | | | | | |

【Figure 1b】

【Figure 1c】

Chemical shift (ppm)

【Figure 1d】

CA-SA

Chemical shift (ppm)

【Figure 1e】

【Figure 2】

【Figure 3a】

* comparison between groups compared to not applied
† comparison between groups compared to vehicle-applied
* † p<0.05

【Figure 3b】

* comparison between groups compared to not applied
† comparison between groups compared to vehicle-applied
* † p<0.05, ** †† p<0.01

| INTERNATIONAL SEARCH REPORT | International application No. |
|---|---|
| | **PCT/KR2022/003302** |

**A.   CLASSIFICATION OF SUBJECT MATTER**

**A61K 8/368**(2006.01)i; **A61K 8/44**(2006.01)i; **A61Q 19/00**(2006.01)i; **A61Q 17/00**(2006.01)i

According to International Patent Classification (IPC) or to both national classification and IPC

**B.   FIELDS SEARCHED**

Minimum documentation searched (classification system followed by classification symbols)

A61K 8/368(2006.01); A61K 31/385(2006.01); A61K 31/66(2006.01); A61K 8/36(2006.01); A61K 8/41(2006.01); A61K 8/44(2006.01); A61K 8/97(2006.01); A61Q 19/02(2006.01); A61Q 5/00(2006.01); C07C 229/12(2006.01); C07C 69/157(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 카르니틴(carnitine), 살리실레이트(salicylate), 피부(skin), 화장료(cosmetic composition)

**C.   DOCUMENTS CONSIDERED TO BE RELEVANT**

| Category* | Citation of document, with indication, where appropriate, of the relevant passages | Relevant to claim No. |
|---|---|---|
| A | KR 10-1017586 B1 (AMOREPACIFIC CORPORATION) 28 February 2011 (2011-02-28)<br>      See claims 1-5; and paragraphs [0017]-[0025]. | 1-15 |
| A | JP 2014-114289 A (SHOWA DENKO KK) 26 June 2014 (2014-06-26)<br>      See entire document. | 1-15 |
| A | KR 10-2012-0050437 A (TETRA, SIA) 18 May 2012 (2012-05-18)<br>      See entire document. | 1-15 |
| A | KR 10-2013-0018739 A (NEOCUTIS SA) 25 February 2013 (2013-02-25)<br>      See entire document. | 1-15 |
| A | KR 10-2013-0014519 A (AMOREPACIFIC CORPORATION) 07 February 2013 (2013-02-07)<br>      See entire document. | 1-15 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 June 2022** | **15 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2022/003302**

| Patent document cited in search report | | | Publication date (day/month/year) | Patent family member(s) | | | Publication date (day/month/year) |
|---|---|---|---|---|---|---|---|
| KR | 10-1017586 | B1 | 28 February 2011 | KR | 10-2010-0060711 | A | 07 June 2010 |
| JP | 2014-114289 | A | 26 June 2014 | None | | | |
| KR | 10-2012-0050437 | A | 18 May 2012 | BR | PI1009600 | A2 | 22 March 2016 |
| | | | | CA | 2766048 | A1 | 29 December 2010 |
| | | | | CN | 102803199 | A | 28 November 2012 |
| | | | | CN | 102803199 | B | 15 April 2015 |
| | | | | EA | 021588 | B1 | 30 July 2015 |
| | | | | EA | 201200038 | A1 | 30 October 2012 |
| | | | | EP | 2445861 | A1 | 02 May 2012 |
| | | | | EP | 2445861 | B1 | 26 March 2014 |
| | | | | HK | 1169648 | A1 | 01 February 2013 |
| | | | | JP | 2012-531408 | A | 10 December 2012 |
| | | | | JP | 5706409 | B2 | 22 April 2015 |
| | | | | US | 2012-0088742 | A1 | 12 April 2012 |
| | | | | US | 8889902 | B2 | 18 November 2014 |
| | | | | WO | 2010-151095 | A1 | 29 December 2010 |
| | | | | WO | 2010-151095 | A8 | 29 December 2010 |
| | | | | ZA | 201200592 | B | 26 September 2012 |
| KR | 10-2013-0018739 | A | 25 February 2013 | CA | 2790682 | A1 | 09 September 2011 |
| | | | | CA | 2790682 | C | 24 November 2020 |
| | | | | CN | 102985091 | A | 20 March 2013 |
| | | | | CN | 102985091 | B | 23 November 2016 |
| | | | | EP | 2542246 | A1 | 09 January 2013 |
| | | | | JP | 2013-521300 | A | 10 June 2013 |
| | | | | US | 2011-0217249 | A1 | 08 September 2011 |
| | | | | US | 9629856 | B2 | 25 April 2017 |
| | | | | WO | 2011-109469 | A1 | 09 September 2011 |
| KR | 10-2013-0014519 | A | 07 February 2013 | CN | 102821742 | A | 12 December 2012 |
| | | | | JP | 2013-523721 | A | 17 June 2013 |
| | | | | US | 2013-0018020 | A1 | 17 January 2013 |
| | | | | WO | 2011-122840 | A2 | 06 October 2011 |
| | | | | WO | 2011-122840 | A3 | 01 March 2012 |

Form PCT/ISA/210 (patent family annex) (July 2019)

**EP 4 306 100 A1**

**Patent documents cited in the description**

- KR 1020100095447
- KR 102307186
- KR 1020170076091